# EUROPEAN PATENT SPECIFICATION

(11) **EP 3 441 897 B1**
(45) Date of publication and mention of the grant of the patent: **20.09.2023**
(21) Application number: 17209092.0
(22) Date of filing: 20.12.2017
(51) Int. Cl.: G16H 50/70, G16H 30/40, G16B 40/10

(54) **METHOD AND DEVICE FOR IDENTIFYING CANCER**
VERFAHREN UND VORRICHTUNG ZUR IDENTIFIZIERUNG VON KREBS
PROCÉDÉ ET DISPOSITIF D'IDENTIFICATION DU CANCER

(30) Priority: 11.08.2017 IN 201741028668
(43) Date of publication of application: 13.02.2019
(73) Proprietor: Wipro Limited, 560 035 Karnataka (IN)
(72) Inventor: DAS, Rishav, 711106 West Bengal (IN); MUDI, Sourav, 713427 West Bengal (IN)
(74) Representative: Finnegan Europe LLP

(56) References cited:
- US-A1- 2002 165 837
- DALE BAILEY ET AL: "Nuclear Medicine: from Photons to Physiology", CURRENT PHARMACEUTICAL DESIGN, vol. 9, no. 11, 1 April 2003 (2003-04-01), pages 903-916, XP055485032, NL ISSN: 1381-6128, DOI: 10.2174/1381612033455224
- HAL O ANGER: "Scintillation Camera", THE REVIEW OF SCIENTIFIC INSTRUMENTS,, vol. 29, no. 1, 1 January 1958 (1958-01-01), pages 27-33, XP001428265,

## Description

### Technical Field

This disclosure relates generally to identifying cancer and more particularly to a method and device for identifying cancer.

### Background

Cancer is the leading cause of increased mortality rate worldwide. Early detection and classification of the type of cancer aids in providing better patient care. There are different types of cancers, for example, skin cancer, bone cancer, or sarcoma cancer. Multiple techniques are currently employed to screen patients for the above mentioned types of cancer. These techniques, for example, include computed tomography, magnetic resonance imaging, Positron Emission Tomography (PET), ultrasound examination, or mammography. However, not only are these techniques very expensive, the images thus formed require a professional analysis in order to identify the type of cancer cell. This manual classification is a time consuming task and is highly susceptible to human errors.

A conventional system for detecting cancer includes a radiation receiving device, an associated signal processor, and an analyzer. However, this conventional system requires the need to take images of a patient from different angles and utilizes the output derived from the x-rays emitted from the cells to identify the cancer affected region. The conventional system is thus configured to work with particular types of computing devices, which leads to hardware constraints. As a result, data captured by the conventional system cannot be synced up to any platform which is accessible by multiple physiologists.

There is therefore a need for a system and method which is cost effective and provides accurate results for detection and identification of cancer in a patient.

US2002/165837 describes a method of computer-aided analysis in which digitized image data are input into a processor where a detection component identifies the areas (objects) of particular interest in the image and, by segmentation, separates those objects from the background. A feature extraction component formulates numerical values relevant to the classification task from the segmented objects. Results of the preceding analysis steps are input into a trained learning machine classifier which produces an output which may consist of an index discriminating between two possible diagnoses, or some other output in the desired output format. In one embodiment, digitized image data are input into a plurality of subsystems, each subsystem having one or more support vector machines. Pre-processing may include the use of known transformations which facilitate extraction of the useful data. Each subsystem analyzes the data relevant to a different feature or characteristic found within the image. Once each subsystem completes its analysis and classification, the output for all subsystems is input into an overall support vector machine analyzer which combines the data to make a diagnosis, decision or other action which utilizes the knowledge obtained from the image.

### SUMMARY

One aspect of the present invention provides a cancer identifying device in accordance with claim 1. A further aspect of the present invention provides a computer-implemented method for identifying cancer in accordance with claim 6. Yet another aspect provides a computer-readable storage medium in accordance with claim 8.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles.
**FIG. 1** illustrates an environment in which various embodiments may be employed.
**FIG. 2** is a block diagram illustrating various components within a cancer identifying device, in accordance with an embodiment
**FIG. 3** illustrates a flowchart of a method for identifying cancer in an organism, in accordance with an embodiment.
**FIG. 4** illustrates a flowchart of a method for identifying cancer and depicting cancer affected body parts in an organism, in accordance with an embodiment.
**FIG. 5** illustrates a millimeter graph, in accordance with an exemplary embodiment.
**FIGs. 6A** **and** **6B** illustrate identification of cancer affected areas in a person's body, when cancer cells are scattered, in accordance with an exemplary embodiment.
**FIG. 7** illustrates identification of cancer affected areas in a person's body when cancer cells exist in a mass form, in accordance with an exemplary embodiment
**FIG. 8** illustrates a flowchart of a method for providing a treatment track for a cancer affected organism over a period of time, in accordance with an embodiment.
**FIG. 9** illustrates a flowchart of a method for converting gamma photons received through photoreceptor cells to a plurality of oscillating waves, in accordance with an embodiment.
**FIG. 10** illustrates a block diagram of an exemplary computer system for implementing various embodiments.

### DETAILED DESCRIPTION

Exemplary embodiments are described with reference to the accompanying drawings. Wherever convenient, the same reference numbers are used throughout the drawings to refer to the same or like parts. While examples and features of disclosed principles are described herein, modifications, adaptations, and other implementations are possible without departing from the scope of the disclosed embodiments.

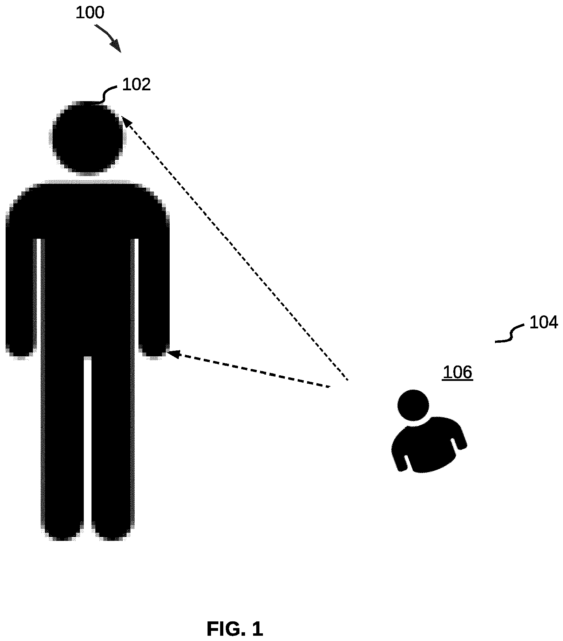

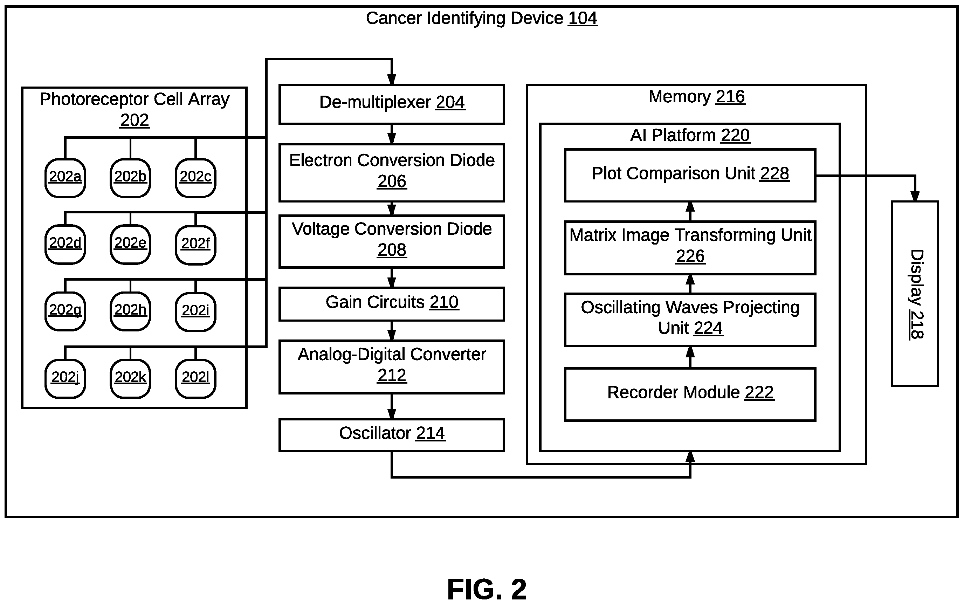

Additional illustrative embodiments are listed below. In one embodiment, FIG. 1 illustrates an exemplary environment 100 in which various embodiments may be employed. Environment 100 includes a person 102, who is being scanned using a cancer identifying device 104. It will be apparent to a person skilled in the art that person 102 may be replaced by any living organism that may be susceptible to various types of cancers. Cancer identifying device 104, for example, may include, but is not limited to a smart phone, a tablet, or a specialized mobile device equipped with requisite components as explained in conjunction with FIG. 2.

In order to detect cancer and identify the type of cancer, radioactive tracers or radioactive labels are introduced into the body of person 102 through parenteral injection. It will be apparent to a person skilled in the art that other means of introducing the radioactive tracers may also be used. The radioactive tracers tag to cancer cells within body of person 102 and produce gamma photons that have different frequencies. Photoreceptor cells in cancer identifying device 104 detect and receive these gamma photons. In order to accurately receive each gamma photon emitted from cancer cells within body of person 102, cancer identifying device 104 may scan whole body of person 102 in one go. Alternatively, cancer identifying device 104 may separately scan specific body parts or body portions of person 102. By way of an example, a display 106 of cancer identifying device 104 depicts that the head and torso of person 102 is being scanned.

Person 102 may be manually scanned by another user via cancer identifying device 104. Alternatively, a mechanical apparatus controlling the scanning operation of cancer identifying device 104 may vertically or horizontally move cancer identifying device 104 after every predefined time interval, such that, whole body of person 102 is scanned in portions or only a desired body portion of person 102 is scanned.

Referring now to FIG. 2, a block diagram of various components within cancer identifying device 104 is illustrated, in accordance with an embodiment. Cancer identifying device 104 includes a photoreceptor cells array 202, a de-multiplexer 204, an electron conversion diode 206, a voltage conversion diode 208, gain circuits 210, an analog-digital converter 212, an oscillator 214, a memory 216, and a display 218. In an embodiment, cancer identifying device 104 may be replaced by a cancer identification system, such that, photoreceptor cells array 202, de-multiplexer 204, electron conversion diode 206, voltage conversion diode 208, gain circuits 210, analog-digital converter 212, oscillator 214, and display 218 may reside in a mobile device that has a camera equipped with gamma photon capturing unit. In this case, memory 216 that includes an Artificial Intelligence (Al) platform 220, which further includes a recorder module 222, an oscillating waves projecting unit 224, a matrix image transforming unit 226, and a plot comparison unit 228, may reside a computing device that has advanced computational capabilities.

In order to detect and identify cancer, radioactive tracers are introduced into an organism's body through parenteral injection. The radioactive tracers tag to cancer cells present in the organism and produce gamma photons of different frequencies. The gamma photons are received by one or more of photoreceptor cells 202a to 202I in photoreceptor cells array 202, which is positioned at a minimum distance from the organism's body. Each of photoreceptor cells 202a to 202I are communicatively coupled to de-multiplexer 204 that is configured to accept inputs from those photoreceptor cells triggered by the gamma photons. Electron conversion diode 206 then converts the gamma photons into electrons, which are further converted into voltage signals by voltage conversion diode 208.

As the gamma photons received from the organism's body are not very powerful, gain circuits 210 amplifies the voltage signals. Analog-digital converter 212, then converts the amplified voltage signals to digital signals. Oscillator 214 converts the digital signals into a plurality of oscillating waves of different frequencies, such that, each oscillating wave is associated with a gamma photon emitted by a cancer cell. Oscillator 214 also aids in maximizing the frequency and helps in detecting the difference between normal and cancer effected frequencies.

Oscillator 214 then provides the plurality of oscillating waves to AI platform 220 in memory 216. Memory 216 includes instructions stored thereon, that cause a processor, on execution to identify cancer. Memory 216 may be a non-volatile memory or a volatile memory. Examples of non-volatile memory, may include, but are not limited to a flash memory, a Read Only Memory (ROM), a Programmable ROM (PROM), Erasable PROM (EPROM), and Electrically EPROM (EEPROM) memory. Examples of volatile memory may include, but are not limited Dynamic Random Access Memory (DRAM), and Static Random-Access memory (SRAM).

AI platform 220 is configured to understand and identify various cancerous cells in order to provide treatment track for a cancer affected organism. The AI platform 220 also aids to keep track of patient's data for future research and enables researchers to analyze and research on various cancerous disease. In AI platform 220, recorder module 222 receives the plurality of oscillating waves from oscillator 214. Thereafter, oscillating waves projecting unit 224 creates a contour plot by projecting positive and negative peaks of each of the plurality of oscillating waves. The contour plot is an outline projection of one or more body parts of the organisms that are affected by cancer and thus represents outline of the cancer affected areas.

In an embodiment, matrix image transforming unit 226 transforms the contour plot into a matrix image view in order to convert the contour plot into pixel value. As a result, using the pixel value, the contour plot can be easily stored in memory 216 of cancer identifying device 104. This can be later utilized to assess growth of cancer cells in the organism over a period of time.

Finally, in order to identify the type of cancer affecting one or more body parts of the organism, plot comparison unit 228 compares pattern of the contour plot with a plurality of training contour plots to find a match. Each of the plurality of training contour plots are tagged with an associated type of cancer. In other words, a given training contour plot is tagged with data regarding the type and extent of cancer in that training contour plot. Thus, when the contour plot matches with this training contour plot, it would imply that the organism also suffers from the same type and extent of cancer, as tagged with the training contour plot.

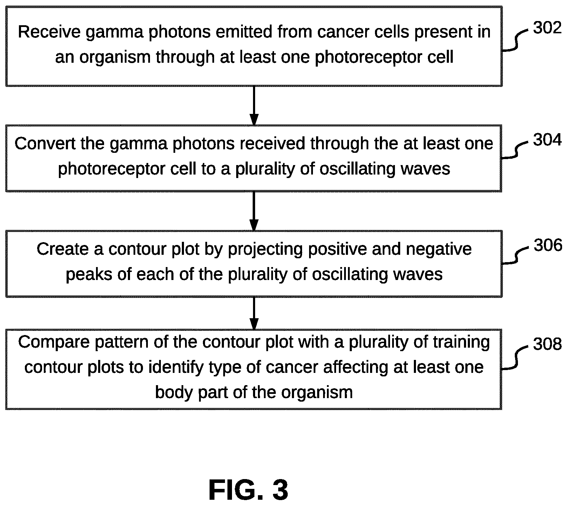

Once the type of cancer affecting one or more body parts of the organism are identified, a graphical image depicting the one or more body parts affected by the type of cancer and indicating degree of spread of the type of cancer within the one or more body parts is displayed on display 218. This is further explained in detail in conjunction with FIG. 3, FIG. 4, and FIG. 8.

Referring now to FIG. 3, a flowchart of a method for identifying cancer in an organism is illustrated, in accordance with an embodiment. The organism, for example, may include, but is not limited to a human or an animal. In order to detect and identify cancer, radioactive tracers are introduced into the organism's body through parenteral injection. The radioactive tracers tag to cancer cells present in the organism and produce gamma photons of different frequencies.

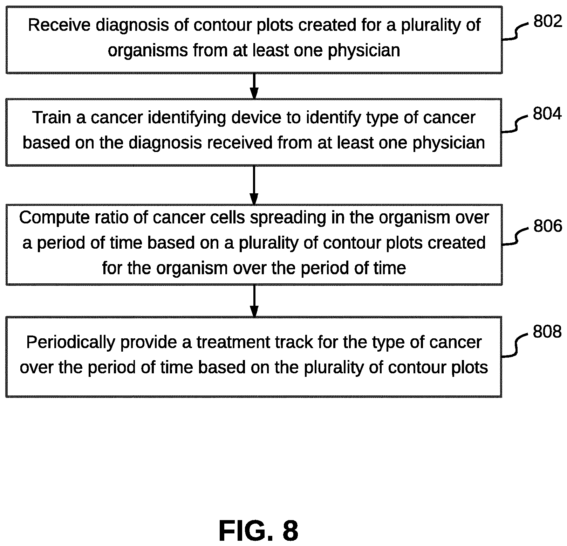

At step 302, cancer identifying device 104, receives the gamma photons emitted from the cancer cells, through one or more photoreceptor cells in photoreceptor cells array 202. At step 304, cancer identifying device 104 converts the gamma photons to a plurality of oscillating waves that have a plurality of frequencies. In other words, each oscillating wave has a different frequency and represents one gamma photon. The conversion of the gamma photons to the plurality of oscillating waves is explained in detail in conjunction with FIG. 8. An oscillating wave may either be an asynchronous oscillating wave or a synchronous oscillating wave (for example, a sinusoidal wave). An asynchronous oscillating wave is associated with a gamma photon emitted from a cancer cell, while a synchronous oscillating wave is associated with a gamma photon emitted by a healthy cell that incudes glucose. Cancer identifying device 104 is trained to ignore the synchronous oscillating waves and only processes asynchronous oscillating waves.

At step 306, cancer identifying device 104 creates a contour plot by projecting positive and negative peaks of each of the plurality of oscillating waves. Cancer identifying device 104 uses oscillating waves projecting unit 224 to create the contour plot. The contour plot is an outline projection of one or more body parts of the organisms that are affected by cancer and thus represents outline of the cancer affected areas.

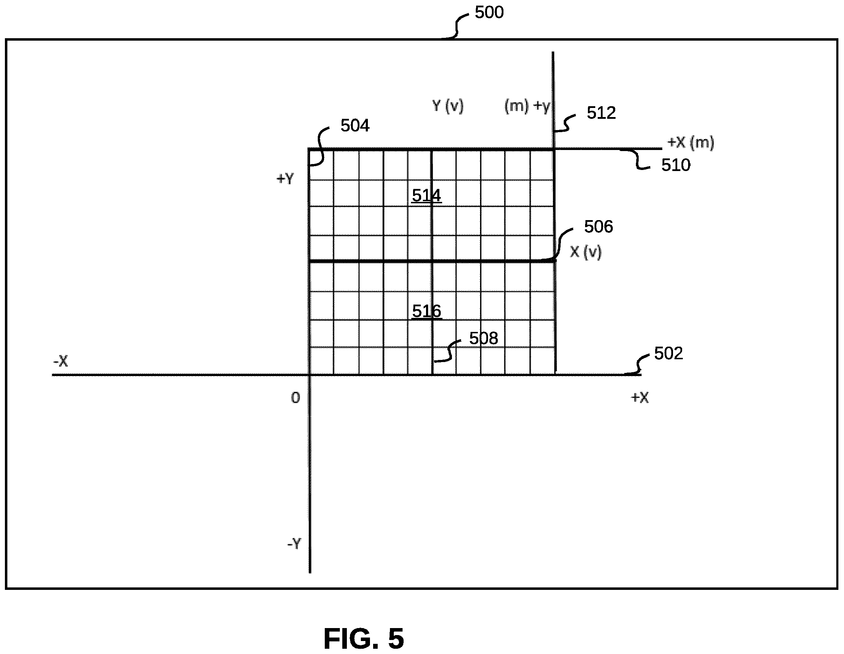

In an embodiment, the contour plot is projected on a millimeter graph that includes a positive-positive quadrant. The millimeter graph includes a virtual X plane (represented as: X (v)) and a virtual Y plane (represented as: Y (v)). In these virtual planes, (v) is a predefined function of one or more parameters, which include: a positive peak (or positive penetration) value of an oscillating wave, a negative peak (or negative penetration) value of the oscillating wave, distance covered by the gamma photon (associated with the oscillating wave) to reach photoreceptor cells array 202, and time taken by the gamma photon to be detected by photoreceptor cells array 202. The intersection of X (v) and Y (v) divides the graph into an upper plane and a lower plane. This is clearly depicted in an exemplary millimeter graph 500 illustrated in FIG. 5.

The one or more parameters help in determining which portions of the organism's body are affected by cancer and by which type of cancer. The parameters of positive peak (or positive penetration) and negative peak (or negative penetration) are used to determine whether cancer cells are growing in an object oriented form (i.e., affecting an organ) or in a linear or scattered form within the organism. The scattering of cancer cells may either be localized or may have spread across whole body of the organism. An oscillating wave (associated with a gamma photon) that has both negative peaks and positive peaks indicates that the cancer cell emitting the gamma photon exists within a mass form. In other words, the cancer cell is part of an organ affected by cancer.

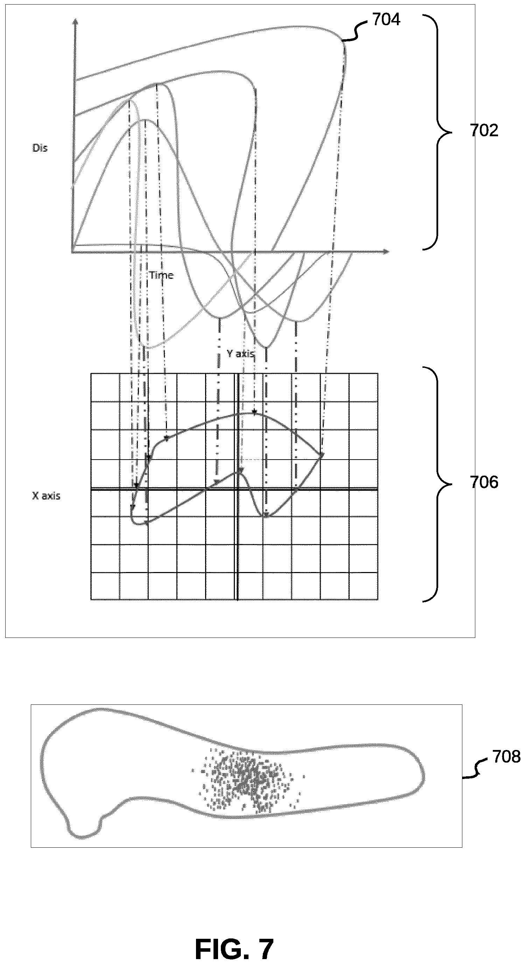

For one or more oscillating waves that include both positive peaks and negative peaks, the positive peaks are plotted or projected in the upper plane of the positive-positive quadrant and the negative peaks are plotted or projected in the lower plane of the positive-positive quadrant. This is further explained in detail in conjunction with the exemplary embodiment of FIG. 7. By way of an example, if the cancer cells form a tumor, then the plot will be volume or object oriented and would be projected on both the upper place and the lower plane. Moreover, when an object is captured as cancer affected area, a positive penetration of an oscillating wave would indicate existence of a cancer cell on front part of the object and negative penetration of an oscillating wave would indicate existence of a cancer cell on back part of the object. This would help in identifying orientation of the object within the body.

Objects formed in a contour plot may ideally be centered around an intersection of the virtual X plane and the virtual Y plane of the millimeter graph. However, based on the position of cancer identifying device 104 scanning the organism, an object that appears on the contour plot may be offset from the intersection. In this case, positioning of cancer identifying device 104 may have to be adjusted in order to bring an object close to the intersection on the millimeter graph. In another scenario, multiple objects may appear on the contour plot and each of these objects may be offset from the intersection. In this case, positioning of cancer identifying device 104 may have to be adjusted in order to focus only on specific areas of the body, to capture a single object.

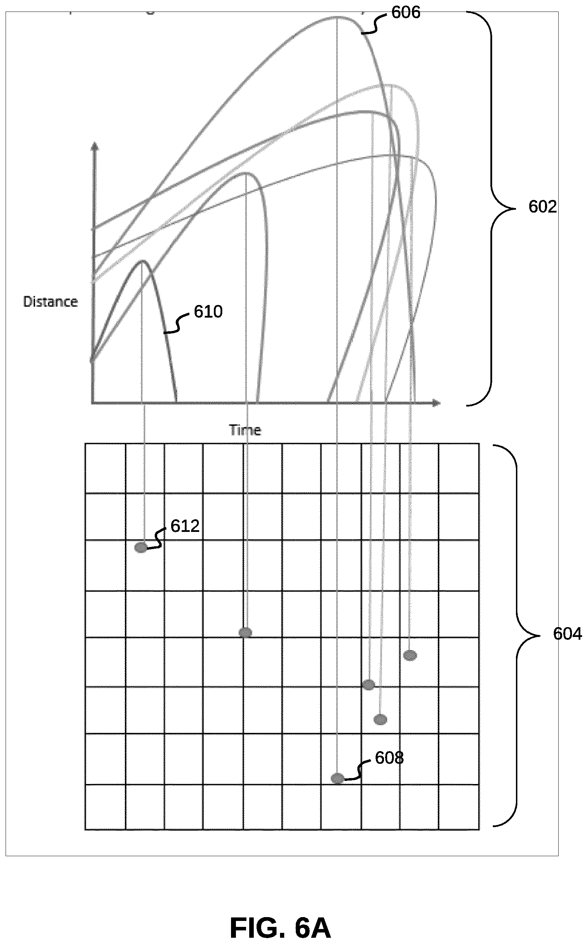

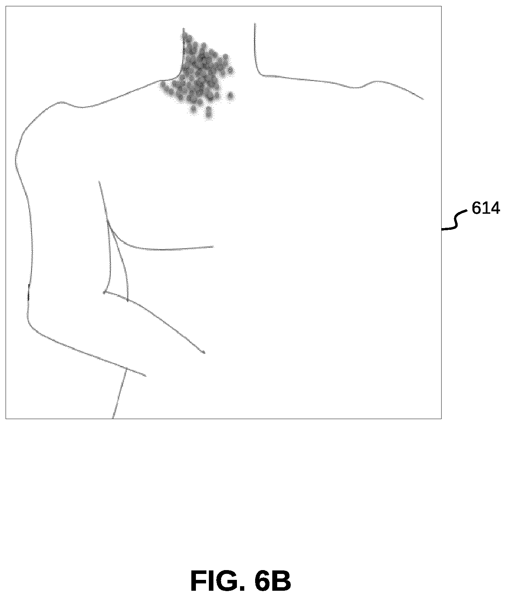

However, an oscillating wave that either has only positive peaks or only negative peaks indicates that the cancer cell is scattered in blood or in skin of the organism. In this case, for one or more oscillating waves that include only positive peaks or only negative peaks, both positive peaks and the negative peaks would be plotted in the upper plane of the positive-positive quadrant. This is further explained in detail in conjunction with the exemplary embodiment of FIG. 6A and 6B. By way of an example, if the cancer cells are underlying the skin, then the contour plot would be projected only on the upper plane.

In order to clearly identify location of the cancer affected areas, the amplitude and wavelength of each of the plurality of oscillating waves is analyzed. The body of an organism may be divided into three parts: front lobe (for example, skin, i.e., epidermis, dermis, or hypodermis, or blood), middle lobe (for example, organs), and back lobe (for example, skin or blood). Amplitude of an oscillating wave is directly proportional to the distance travelled by an associated gamma photon from a cancer affected cell to reach a photoreceptor cell in photoreceptor cells array 202. In other words, higher is the amplitude of an oscillating wave, greater is the distance travelled by an associated gamma photon. Additionally, wavelength of an oscillating wave is directly proportional to the time taken by an associated gamma photon to reach a photoreceptor cell. In other words, greater is the wavelength of an oscillating wave, more is the time taken by an associated gamma photon to reach photoreceptor cells array 202.

Thus, oscillating waves that have high amplitude with big wavelength indicate that the cancer affected area is located in the back lobe of the body. Similarly, oscillating waves that have low amplitude with small wavelength indicate that the cancer affected area is located in the frontal lobe of the body. Oscillating waves that have high amplitude and small wavelength indicate that the cancer affected area are within internal layers of the body. Similarly, oscillating waves that have low amplitude and big wavelength indicate that cancer affected area is within an organ (located in the middle lobe) of the organism.

In an embodiment, in order to determine whether an amplitude or wavelength is high, medium, or low, multiple thresholds may be automatically defined and adjusted based on the range of amplitudes or wavelength produced by oscillator 214. The plotting of contours on the millimeter graph is further illustrated in detail in conjunction with exemplary embodiments of FIG. 6A, FIG. 6B, and FIG. 7.

At step 308, cancer identifying device 104 compares pattern of the contour plot with a plurality of training contour plots in order to identify type of cancer affecting one or more body parts of the organism. Plot comparison unit 228 in cancer identifying device 104 may perform the comparison in order to find a match from the plurality of training contour plots. Each of the plurality of training contour plots are tagged with an associated type of cancer. In other words, a given training contour plot is tagged with data regarding the type and extent of cancer in that training contour plot.

Thus, when the contour plot created by cancer identifying device 104 matches with this training contour plot, it would imply that the organism also suffers from the same type and extent of cancer, as tagged with the training contour plot. By way of an example, if the contour plot appears to be in distributed format from an affected region or from the whole body, it may be inferred that epithelial cells are affected and the organism is suffering from skin cancer. By way of another example, when the gamma photon concentration is mostly emitted from connective tissue, inference can be made that Sarcoma cancer cells are present. By way of yet another example, if the contour plot projects an image depicting outline of bone like structure, it can be inferred that the organism is suffering from Leukemia.

In an embodiment, when plot comparison unit 228 is not able to find a match with one of the plurality of training contour plots, cancer identifying device 104 initiates communication with physiologist, doctors, professors, or other experts in this field in order to identify new type of cancer cells. This is further explained in detail in conjunction with FIG. 8.

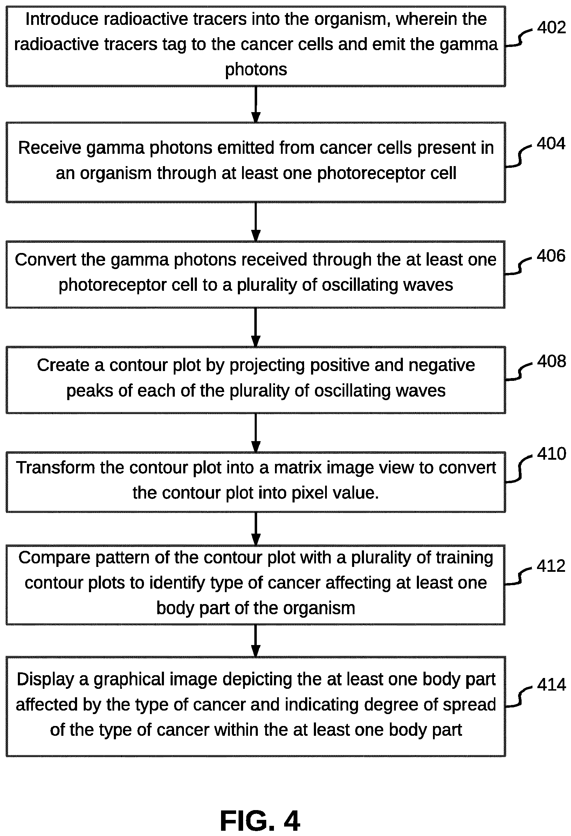

Referring now to FIG. 4, a flowchart of a method for identifying cancer and depicting cancer affected body parts in an organism is illustrated, in accordance with an embodiment. At step 402, radioactive tracers are introduced into the organism by way of a parenteral injection. Once injected, the radioactive tracers tag to cancer cells in the organism and emit gamma photons. At step 404, the gamma photons emitted from the cancer cells are received through one or more photoreceptor cells in photoreceptor cells array 202. At step 406, the gamma photons received through the one or more photoreceptor cells are converted to a plurality of oscillating waves. This is further explained in detail in conjunction with FIG. 8.

At step 408, a contour plot is created by projecting positive and negative peaks of each of the plurality of oscillating waves on a millimeter graph. This has already been explained in detail in conjunction with FIG. 3. At step 410, the contour plot is transformed into a matrix image view in order to convert the contour plot into pixel value. As a result, using the pixel value, the contour plot can be easily stored in memory 216 of cancer identifying device 104. This can be later utilized to assess growth of cancer cells in the organism over a period of time. Matrix image transforming unit 226 may be used to transform the contour plot.

Thereafter, at step 412, pattern of the contour plot is compared with a plurality of training contour plots in order to identify type of cancer affecting one or more body parts of the organism. This has already been explained in detail in conjunction with FIG. 3. In response to finding a match with one or more of the plurality of training contour plots or based on comments received from a cancer expert, at step 414, a graphical image depicting the one or more body parts affected by the type of cancer and indicating degree of spread of the type of cancer within the one or more body parts is displayed. The graphical image is displayed on display 218 of cancer identifying device 104 or on display of a computing device in communication with cancer identifying device 104. Exemplary graphical images depicting cancer affected areas and the type of cancer are illustrated in FIG. 6B and FIG. 7.

Referring now to FIG. 5, millimeter graph 500 is illustrated, in accordance with an exemplary embodiment. It will be apparent to a person skilled in the art that millimeter graph 500 is not drawn to scale for ease of explanation. Millimeter graph 500 incudes the positive-positive quadrant. In other words, millimeter graph 500 is drawn in the quadrant that has a positive X plane 502 and a positive Y plane 504. Within the positive-positive quadrant, millimeter graph 500 includes a virtual X plane 506 (represented as: X (v)), a virtual Y plane 508 (represented as: Y (v)), a mirrored positive X plane 510 (represented as: X (m)), and a mirrored positive Y plane 512 (represented as: Y (m)).

Thus, only the area enclosed within positive X plane 502, mirrored positive X plane 510, positive Y plane 504, and mirrored positive Y plane 512 is considered for creating the contour plot and the remaining portion of millimeter graph 500 is not used. Moreover, the intersection of virtual X plane 506 and virtual Y plane 508 creates an upper plane 514 and a lower plane 516. In other words, millimeter graph 500 includes equalized quadrants and each of the quadrants in millimeter graph 500 includes positive x and y planes. As a result, no portion of the contour plot is projected in a negative plane.

Referring now to FIGs. 6A and 6B, identification of cancer affected areas in a person's body when cancer cells are scattered, is illustrated, in accordance with an exemplary embodiment. An oscillator output graph 602 depicts multiple oscillating waves that are plotted on a distance-time graph, such that Y plane represent distance, while X plane represents time. Each oscillating wave in oscillator output graph 602 represents a gamma photon emitted by a cancer cell. It will apparent to a person skilled in the art that limited number of oscillating waves are depicted in oscillator output graph 602 for ease of explanation.

Each oscillating wave in oscillator output graph 602 has a positive peak or positive penetration only. In other words, the cancer cells emitting the gamma photons are scattered within a particular body portion of the person. The scattering of the cancer cells is depicted by projecting a contour plot on a millimeter graph 604, which is depicting only the upper plane of a positive-positive quadrant. In oscillator output graph 602, an oscillating wave 606 has the highest amplitude and biggest wavelength. In other words, a gamma photon associated with oscillating wave 606 has covered the maximum distance and has taken the maximum time to reach photoreceptor cells array 202 from a cancer cell. Thus, the cancer cell is located farthest from the photoreceptor cells array 202 in the body of the person (either back lobe or middle lobe) and is also projected farthest on millimeter graph 604 at point 608.

In contrast, an oscillating wave 610 has the lowest amplitude and the least wavelength. A gamma photon associated with oscillating wave 610 has covered the minimum distance and has taken the least time to reach photoreceptor cells array 202 from a cancer cell. Thus, the cancer cell is located closest from the photoreceptor cells array 202 in the body of the person (front lobe) and is also projected closest on millimeter graph 604 at point 612. It is apparent from the contour plot on millimeter graph 604 that the points projected on millimeter graph 604 are scattered and do not form a definite outline or shape.

This contour plot is then compared with a plurality of training contour plots in order to find a match and identify the type of cancer affecting the person. The contour plot may match with a training contour plot that is tagged with lymph node cancer. Thus, cancer identifying device 104 displays a graphical image 614 depicting that the person is suffering from lymph node cancer around the neck area. Graphical image 614 may be displayed to a doctor, an administrator, or the person himself/herself indicating the affected body portion of the person. Though not illustrated, graphical image 614 may also include details regarding the type of cancer and recommendation as to the treatment procedure required to treat the cancer.

Referring now to FIG. 7, identification of cancer affected areas in a person's body when cancer cells exist in a mass form, is illustrated, in accordance with an exemplary embodiment. An oscillator output graph 702 depicts multiple oscillating waves that are plotted on a distance-time graph, such that Y plane represent distance, while X plane represent time. Each oscillating wave in oscillator output graph 702 represents a gamma photon emitted by a cancer cell. It will apparent to a person skilled in the art that limited number of oscillating waves are depicted in oscillator output graph 702 for ease of explanation.

Only one oscillating wave, i.e., an oscillating wave 704, in oscillator output graph 702 has only a positive peak or positive penetration. Other oscillating waves in oscillator output graph 702 have both positive peaks and negative peaks. This indicates that the cancer cells are centered around a mass or within an organ and also that cancer cells are scattered within the organ. In other words, though an organ is affected by cancer, cancer cells within the organ are scattered. Formation of the mass is depicted by an outline of the mass projected on a millimeter graph 706 based on the oscillating waves in oscillator output graph 702.

Oscillating wave 704 has the highest amplitude, biggest wavelength, and only positive penetration, thus it indicates that a cancer cell associated with oscillating wave 704 is scattered within the organ at farther end of the organ, when compared with location of photoreceptor cells array 202. This contour plot is then compared with a plurality of training contour plots in order to find a match and identify the type of cancer affecting the person. The contour plot may match with a training contour plot that is tagged with pancreatic cancer. Thus, cancer identifying device 104 displays a graphical image 708 depicting that the person is suffering from pancreatic cancer. Graphical image 708 also depicts that the cancer cells are densely scattered at center of the pancreas and are sparsely scattered toward right and left side of this central concentration. As a result, a person viewing graphical image 708 can easily identify the area within the pancreas that has a higher concentration of cancer cells and the direction in which the cancer cells are spreading within the pancreas.

Referring now to FIG. 8, a flowchart of a method for providing a treatment track for a cancer affected organism over a period of time is illustrated, in accordance with an embodiment. At step 802, diagnosis of contour plots created for a plurality of organisms are received from one or more physicians. The contour plots may be shared on a platform that is accessed by one or more physicians, doctors, professors, or other experts, who may provide their diagnosis of the contour plots and required treatment to treat the diagnosed cancer. Based on this diagnosis and a plurality of training contour plots, cancer identifying device 104 is trained, at step 804, to identify type of cancer in a contour plot created for the cancer affected organism injected with radioactive tracers.

At step 806, cancer identifying device 104 computes ratio of cancer cells spreading in the cancer affected organism over a period of time is based on a plurality of contour plots created for the cancer affected organism over the period of time. To this end, cancer identifying device 104 calculates the ratio of number of oscillating curves that have only positive peak or only negative peaks over a period of time. This helps in determining the amount of cancer cell scattering that has happened over the period of time. Higher is the amount of cancer cell scattering over a period of time, higher is the rate of spreading of cancer in the organism. This data associated with the cancer affected organism is also stored for future analysis and training.

Based on the training and the plurality of contour plots diagnosed by the one or more physicians, cancer identifying device 104 periodically provides a treatment track for the type of cancer over the period of time at step 808. The rate of spreading of the cancer may also be considered in order to modify the treatment track over the period of time. The treatment track may be provided to an end user, via display 218.

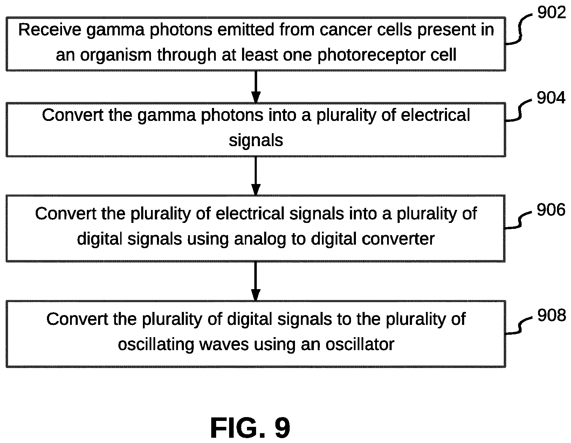

Referring now to FIG. 9, a flowchart of a method for converting gamma photons received through photoreceptor cells to a plurality of oscillating waves is illustrated, in accordance with an embodiment. At step 902, gamma photons emitted from cancer cells present in an organism are received through one or more photoreceptor cells in photoreceptor cells array 202. Thereafter, at step 904, gamma photons are converted into a plurality of electrical signals using converter elements, such as, electron conversion diode 206 and voltage conversion diode 208. At step 906, the plurality of electrical signals is converted into a plurality of digital signals using analog-digital converter 212. Thereafter, at step 908, the plurality of digital signals is converted into a plurality of oscillating waves using oscillator 214. Oscillator 214 also aids in maximizing the frequency of the plurality of digital signals and helps in detecting the difference between normal and cancer effected frequencies. The plurality of oscillating waves is then transmitted to recorder module 222. This has already been explained in detail in conjunction with FIG. 2.

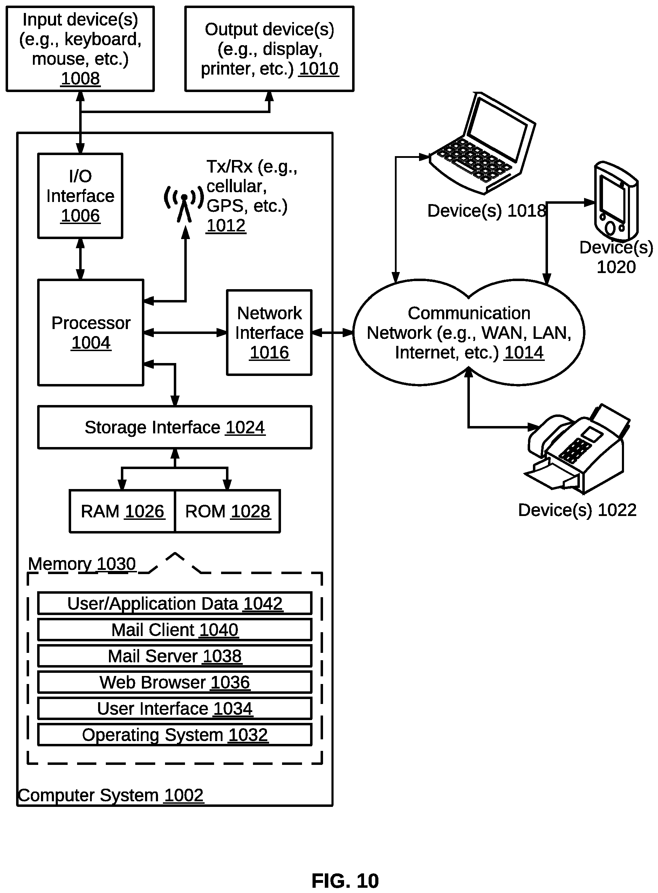

Referring now to FIG. 10, a block diagram of an exemplary computer system for implementing various embodiments is illustrated. Computer system 1002 may include a central processing unit ("CPU" or "processor") 1004 that includes at least one data processor for executing program components for executing user-generated requests or system-generated requests. A user may include a person, a person using a device such as such as those included in this disclosure, or such a device itself. Processor 1004 may include specialized processing units such as integrated system (bus) controllers, memory management control units, floating point units, graphics processing units, digital signal processing units, etc. Processor 1004 may include a microprocessor, such as AMD^{®} ATHLON^{®} microprocessor, DURON^{®} microprocessor OR OPTERON^{®} microprocessor, ARM's application, embedded or secure processors, IBM^{®} POWERPC^{®}, INTEL'S CORE^{®} processor, ITANIUM^{®} processor, XEON^{®} processor, CELERON^{®} processor or other line of processors, etc. Processor 1004 may be implemented using mainframe, distributed processor, multi-core, parallel, grid, or other architectures. Some embodiments may utilize embedded technologies like application-specific integrated circuits (ASICs), digital signal processors (DSPs), Field Programmable Gate Arrays (FPGAs), etc.

Processor 1004 may be disposed in communication with one or more input/output (I/O) devices via an I/O interface 1006. I/O interface 1006 may employ communication protocols/methods such as, without limitation, audio, analog, digital, monoaural, RCA, stereo, IEEE-1394, serial bus, universal serial bus (USB), infrared, PS/2, BNC, coaxial, component, composite, digital visual interface (DVI), high-definition multimedia interface (HDMI), RF antennas, S-Video, VGA, IEEE 802.n /b/g/n/x, Bluetooth, cellular (e.g., code-division multiple access (CDMA), high-speed packet access (HSPA+), global system for mobile communications (GSM), long-term evolution (LTE), WiMax, or the like), etc.

Using I/O interface 1006, computer system 1002 may communicate with one or more I/O devices. For example, an input device 1008 may be an antenna, keyboard, mouse, joystick, (infrared) remote control, camera, card reader, fax machine, dongle, biometric reader, microphone, touch screen, touchpad, trackball, sensor (e.g., accelerometer, light sensor, GPS, gyroscope, proximity sensor, or the like), stylus, scanner, storage device, transceiver, video device/source, visors, etc. An output device 1010 may be a printer, fax machine, video display (e.g., cathode ray tube (CRT), liquid crystal display (LCD), light-emitting diode (LED), plasma, or the like), audio speaker, etc. In some embodiments, a transceiver 1012 may be disposed in connection with processor 1004. Transceiver 1012 may facilitate various types of wireless transmission or reception. For example, transceiver 1012 may include an antenna operatively connected to a transceiver chip (e.g., TEXAS^{®} INSTRUMENTS WILINK WL1283^{®} transceiver, BROADCOM^{®} BCM4550IUB8^{®} transceiver, INFINEON TECHNOLOGIES^{®} X-GOLD 618-PMB9800^{®} transceiver, or the like), providing IEEE 802.11a/b/g/n, Bluetooth, FM, global positioning system (GPS), 2G/3G HSDPA/HSUPA communications, etc.

In some embodiments, processor 1004 may be disposed in communication with a communication network 1014 via a network interface 1016. Network interface 1016 may communicate with communication network 1014. Network interface 1016 may employ connection protocols including, without limitation, direct connect, Ethernet (e.g., twisted pair 50/500/5000 Base T), transmission control protocol/internet protocol (TCP/IP), token ring, IEEE 802.11a/b/g/n/x, etc. Communication network 1014 may include, without limitation, a direct interconnection, local area network (LAN), wide area network (WAN), wireless network (e.g., using Wireless Application Protocol), the Internet, etc. Using network interface 1016 and communication network 1014, computer system 1002 may communicate with devices 1018, 1020, and 1022. The devices may include, without limitation, personal computer(s), server(s), fax machines, printers, scanners, various mobile devices such as cellular telephones, smartphones (e.g., APPLE^{®} IPHONE^{®} smartphone, BLACKBERRY^{®} smartphone, ANDROID^{®} based phones, etc.), tablet computers, eBook readers (AMAZON^{®} KINDLE^{®} ereader, NOOK^{®} tablet computer, etc.), laptop computers, notebooks, gaming consoles (MICROSOFT^{®} XBOX^{®} gaming console, NINTENDO^{®} DS^{®} gaming console, SONY^{®} PLAYSTATION^{®} gaming console, etc.), or the like. In some embodiments, computer system 1002 may itself embody one or more of the devices.

In some embodiments, processor 1004 may be disposed in communication with one or more memory devices (e.g., RAM 1026, ROM 1028, etc.) via a storage interface 1024. Storage interface 1024 may connect to memory 1030 including, without limitation, memory drives, removable disc drives, etc., employing connection protocols such as serial advanced technology attachment (SATA), integrated drive electronics (IDE), IEEE-1394, universal serial bus (USB), fiber channel, small computer systems interface (SCSI), etc. The memory drives may further include a drum, magnetic disc drive, magnetooptical drive, optical drive, redundant array of independent discs (RAID), solid-state memory devices, solid-state drives, etc.

Memory 1030 may store a collection of program or database components, including, without limitation, an operating system 1032, user interface application 1034, web browser 1036, mail server 1038, mail client 1040, user/application data 1042 (e.g., any data variables or data records discussed in this disclosure), etc. Operating system 1032 may facilitate resource management and operation of computer system 1002. Examples of operating systems 1032 include, without limitation, APPLE^{®} MACINTOSH^{®} OS X platform, UNIX platform, Unix-like system distributions (e.g., Berkeley Software Distribution (BSD), FreeBSD, NetBSD, OpenBSD, etc.), LINUX distributions (e.g., RED HAT^{®}, UBUNTU^{®}, KUBUNTU^{®}, etc.), IBM^{®} OS/2 platform, MICROSOFT^{®} WINDOWS^{®} platform (XP, Vista/7/8, etc.), APPLE^{®} IOS^{®} platform, GOOGLE^{®} ANDROID^{®} platform, BLACKBERRY^{®} OS platform, or the like. User interface 1034 may facilitate display, execution, interaction, manipulation, or operation of program components through textual or graphical facilities. For example, user interfaces may provide computer interaction interface elements on a display system operatively connected to computer system 1002, such as cursors, icons, check boxes, menus, scrollers, windows, widgets, etc. Graphical user interfaces (GUIs) may be employed, including, without limitation, APPLE^{®} Macintosh^{®} operating systems' AQUA^{®} platform, IBM^{®} OS/2^{®} platform, MICROSOFT^{®} WINDOWS^{®} platform (e.g., AERO^{®} platform, METRO^{®} platform, etc.), UNIX X-WINDOWS, web interface libraries (e.g., ACTIVEX^{®} platform, JAVA^{®} programming language, JAVASCRIPT^{®} programming language, AJAX^{®} programming language, HTML, ADOBE^{®} FLASH^{®} platform, etc.), or the like.

In some embodiments, computer system 1002 may implement a web browser 1036 stored program component. Web browser 1036 may be a hypertext viewing application, such as MICROSOFT^{®} INTERNET EXPLORER^{®} web browser, GOOGLE^{®} CHROME^{®} web browser, MOZILLA^{®} FIREFOX^{®} web browser, APPLE^{®} SAFARI^{®} web browser, etc. Secure web browsing may be provided using HTTPS (secure hypertext transport protocol), secure sockets layer (SSL), Transport Layer Security (TLS), etc. Web browsers may utilize facilities such as AJAX, DHTML, ADOBE^{®} FLASH^{®} platform, JAVASCRIPT^{®} programming language, JAVA^{®} programming language, application programming interfaces (APis), etc. In some embodiments, computer system 1002 may implement a mail server 1038 stored program component. Mail server 1038 may be an Internet mail server such as MICROSOFT^{®} EXCHANGE^{®} mail server, or the like. Mail server 1038 may utilize facilities such as ASP, ActiveX, ANSI C++/C#, MICROSOFT .NET^{®} programming language, CGI scripts, JAVA^{®} programming language, JAVASCRIPT^{®} programming language, PERL^{®} programming language, PHP^{®} programming language, PYTHON^{®} programming language, WebObjects, etc. Mail server 1038 may utilize communication protocols such as internet message access protocol (IMAP), messaging application programming interface (MAPI), Microsoft Exchange, post office protocol (POP), simple mail transfer protocol (SMTP), or the like. In some embodiments, computer system 1002 may implement a mail client 1040 stored program component. Mail client 1040 may be a mail viewing application, such as APPLE MAIL^{®} mail client, MICROSOFT ENTOURAGE^{®} mail client, MICROSOFT OUTLOOK^{®} mail client, MOZILLA THUNDERBIRD^{®} mail client, etc.

In some embodiments, computer system 1002 may store user/application data 1042, such as the data, variables, records, etc. as described in this disclosure. Such databases may be implemented as fault-tolerant, relational, scalable, secure databases such as ORACLE^{®} database OR SYBASE^{®} database. Alternatively, such databases may be implemented using standardized data structures, such as an array, hash, linked list, struct, structured text file (e.g., XML), table, or as object-oriented databases (e.g., using OBJECTSTORE^{®} object database, POET^{®} object database, ZOPE^{®} object database, etc.). Such databases may be consolidated or distributed, sometimes among the various computer systems discussed above in this disclosure. It is to be understood that the structure and operation of the any computer or database component may be combined, consolidated, or distributed in any working combination.

It will be appreciated that, for clarity purposes, the above description has described embodiments of the invention with reference to different functional units and processors. However, it will be apparent that any suitable distribution of functionality between different functional units, processors or domains may be used without detracting from the invention. For example, functionality illustrated to be performed by separate processors or controllers may be performed by the same processor or controller. Hence, references to specific functional units are only to be seen as references to suitable means for providing the described functionality, rather than indicative of a strict logical or physical structure or organization.

Various embodiments of the invention provide method and device for identifying cancer. As a result of dependence on software intelligence, this is a cost effective method for detecting and identifying cancer cells and required less time. Moreover, this invention results in minimum error while detecting, counting, and locating cancer cell. The invention also provides data statics of cancer cell growth and efficiency of medicine in controlling such growth over a period of time. The invention also provides for pre-detective analysis (Ratio of spreading cancer cells), basic treatment guidance, and analysis of different strategic reports (growth of different cancer cells). The invention can be implemented on a portable device and can be used by personnel who is not an expert in the medical field. Moreover, unlike conventional devices, the invention does not capture a patient's image from multiple angles, which requires physical rotation of the organism's body.

The specification has described method and device for identifying cancer. The illustrated steps are set out to explain the exemplary embodiments shown, and it should be anticipated that ongoing technological development will change the manner in which particular functions are performed. Examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

## Claims

1. A cancer identifying device comprising:
a photoreceptor cell array (202) comprising a plurality of photoreceptor cells (202a-202l) and configured to receive gamma photons emitted from cancer cells present in an organism;
means (206) for converting the gamma photons into a plurality of electrical signals;
an analog to digital converter (212) for converting the plurality of electrical signals into a plurality of digital signals;
an oscillator (214) for converting the plurality of digital signals to the plurality of oscillating waves;
a processor communicatively coupled to the photoreceptor cell array; and
a memory (216) communicatively coupled to the processor and having processor instructions stored thereon, which on execution by the processor, cause the processor to:
create (306) a contour plot of a function of the positive and negative peaks of each of the plurality of oscillating waves, wherein the function comprises a predetermined function of one or more parameters which include: the positive peak value of the oscillating wave, the negative peak value of the oscillating wave, the distance covered by the gamma photon associated with the oscillating wave to reach the photoreceptor cell array and the time taken by the gamma photon to be detected by the photoreceptor array, wherein the instructions, on execution, cause the processor to create (306) the contour plot on a millimeter graph comprising a positive-positive quadrant, wherein positive peaks of at least one of the plurality of oscillating waves are plotted in an upper plane of the positive-positive quadrant and negative peaks of at least one of the plurality of oscillating waves are plotted in a lower plane of the positive-positive quadrant; and
compare (308) a pattern of the contour plot with a plurality of training contour plots to identify a type of cancer affecting at least one body part of the organism, wherein each of the plurality of training contour plots are tagged with an associated type of cancer.

2. The cancer identifying device of any of the preceding claims, wherein the processor instructions, on execution, further cause the processor to transform (410) the contour plot into a matrix image view to convert the contour plot into pixel value.

3. The cancer identifying device of any of the preceding claims, wherein the processor instructions, on execution, further cause the processor to train the cancer identifying device to identify a type of cancer by receiving diagnosis of contour plots created for a plurality of organisms from at least one physician.

4. The cancer identifying device of any of the preceding claims, further comprising an oscillating waves projector configured to create the contour plot, wherein the contour plot represents outline of the at least one body part affected by cancer.

5. The cancer identifying device of any of the preceding claims, wherein the photoreceptor cells (202a-202l) are configured to receive gamma photons emitted as a result of introducing radioactive tracers into the organism, wherein the radioactive tracers tag to the cancer cells and emit the gamma photons.

6. A computer-implemented method for identifying cancer comprising:
converting gamma photons into a plurality of electrical signals, wherein the gamma photons emitted from cancer cells present in an organism through at least one photoreceptor cell have been received by a cancer identifying device,
converting the plurality of electrical signals into a plurality of digital signals using analog to digital converter;
converting the plurality of digital signals to the plurality of oscillating waves using an oscillator;
creating (408), by the cancer identifying device, a contour plot of a function of the positive and negative peaks of each of the plurality of oscillating waves wherein the function comprises a predetermined function of one or more parameters which include: the positive peak value of the oscillating wave, the negative peak value of the oscillating wave, the distance covered by the gamma photon associated with the oscillating wave to reach the photoreceptor cell array and the time taken by the gamma photon to be detected by the photoreceptor array, wherein creating (408) the contour plot comprises creating (408) the contour plot on a millimeter graph comprising a positive-positive quadrant, wherein positive peaks of at least one of the plurality of oscillating waves are plotted in an upper plane of the positive-positive quadrant and negative peaks of at least one of the plurality of oscillating waves are plotted in a lower plane of the positive-positive quadrant; and
comparing (412), by the cancer identifying device, pattern of the contour plot with a plurality of training contour plots to identify type of cancer affecting at least one body part of the organism, wherein each of the plurality of training contour plots are tagged with an associated type of cancer.

7. The method of claim 6, further comprising training the cancer identifying device to identify type of cancer by receiving diagnosis of contour plots created for a plurality of organisms from at least one physician.

8. A computer-readable storage medium having instructions stored thereon that, when executed by one or more processors, cause a cancer identifying device to perform the method of claim 6 or claim 7.

## Patentansprüche

1. Krebsidentifizierungsvorrichtung, umfassend:
eine Photorezeptorzellenanordnung (202), umfassend eine Vielzahl von Photorezeptorzellen (202a-202l) und die konfiguriert ist, um Gammaphotonen zu empfangen, die von Krebszellen emittiert werden, die in einem Organismus vorhanden sind;
Einrichtungen (206) zum Umwandeln der Gammaphotonen in eine Vielzahl von elektrischen Signalen;
einen Analog-Digital-Wandler (212) zum Umwandeln der Vielzahl von elektrischen Signale in eine Vielzahl von digitalen Signalen;
einen Oszillator (214) zum Umwandeln der Vielzahl von digitalen Signalen in die Vielzahl von Oszillationswellen;
einen Prozessor, der kommunikativ mit der Photorezeptorzellenanordnung gekoppelt ist; und
einen Speicher (216), der kommunikativ mit dem Prozessor gekoppelt ist und in dem Prozessoranweisungen gespeichert sind, die bei Ausführung durch den Prozessor den Prozessor zu Folgendem veranlassen:
Erstellen (306) eines Konturdiagramms einer Funktion der positiven und negativen Spitzen von jeder der Vielzahl von oszillierenden Wellen, wobei die Funktion eine vorbestimmte Funktion von einem oder einer Vielzahl von Parametern umfasst, die Folgendes beinhalten: den positiven Spitzenwert der oszillierenden Welle, den negativen Spitzenwert der oszillierenden Welle, die Strecke, die das mit der oszillierenden Welle assoziierte Gammaphoton zurücklegt, um die Photorezeptorzellenanordnung zu erreichen, und die Zeit, die das Gammaphoton benötigt, um von der Photorezeptoranordnung detektiert zu werden, wobei die Anweisungen den Prozessor bei Ausführung veranlassen, das Konturdiagramm auf einem Millimetergraphen zu erstellen (306), der einen positiv-positiven Quadranten umfasst, wobei positive Spitzen von mindestens einer der Vielzahl von oszillierenden Wellen in einer oberen Ebene des positiv-positiven Quadranten aufgetragen werden und negative Spitzen von mindestens einer der Vielzahl von oszillierenden Wellen in einer unteren Ebene des positiv-positiven Quadranten aufgetragen werden; und
Vergleichen (308) eines Musters des Konturdiagramms mit einer Vielzahl von Trainingskonturdiagrammen, um eine Krebsart zu identifizieren, die mindestens einen Körperteil des Organismus betrifft, wobei jedes der Vielzahl von Trainingskonturdiagrammen mit einer assoziierten Krebsart markiert ist.

2. Krebsidentifizierungsvorrichtung nach einem der vorherigen Ansprüche, wobei die Prozessoranweisungen den Prozessor bei Ausführung ferner veranlassen, das Konturdiagramm in eine Matrixbildansicht zu transformieren (410), um das Konturdiagramm in Pixelwerte umzuwandeln.

3. Krebsidentifizierungsvorrichtung nach einem der vorherigen Ansprüche, wobei die Prozessoranweisungen bei Ausführung den Prozessor ferner veranlassen, die Krebsidentifizierungsvorrichtung zu trainieren, um eine Krebsart zu identifizieren, indem eine Diagnose von Konturdiagrammen, die für eine Vielzahl von Organismen erstellt werden, von mindestens einem Arzt empfangen wird.

4. Krebsidentifizierungsvorrichtung nach einem der vorherigen Ansprüche, ferner umfassend einen oszillierenden Wellenprojektor, der konfiguriert ist, um das Konturdiagramm zu erstellen, wobei das Konturdiagramm den Umriss des mindestens einen von Krebs betroffenen Körperteils darstellt.

5. Krebsidentifizierungsvorrichtung nach einem der vorherigen Ansprüche, wobei die Photorezeptorzellen (202a-202l) konfiguriert sind, um Gammaphotonen zu empfangen, die als Resultat eines Einbringens radioaktiver Tracer in den Organismus emittiert werden, wobei die radioaktiven Tracer an die Krebszellen anlagern und die Gammaphotonen emittieren.

6. Computer-implementiertes Verfahren zum Identifizieren von Krebs, umfassend:
Umwandeln von Gammaphotonen in eine Vielzahl von elektrischen Signalen, wobei die Gammaphotonen, die von Krebszellen, die in einem Organismus vorhanden sind, durch mindestens eine Photorezeptorzelle emittiert werden, von einer Krebsidentifizierungsvorrichtung empfangen wurden,
Umwandeln der Vielzahl von elektrischen Signalen in eine Vielzahl von digitalen Signalen unter Verwendung eines Analog-Digital-Wandlers;
Umwandeln der Vielzahl von digitalen Signale in die Vielzahl von Oszillationswellen unter Verwendung eines Oszillators;
Erstellen (408), durch die Krebsidentifizierungsvorrichtung, eines Konturdiagramms einer Funktion der positiven und negativen Spitzen jeder der Vielzahl von oszillierenden Wellen, wobei die Funktion eine vorbestimmte Funktion von einem oder einer Vielzahl von Parametern umfasst, die Folgendes beinhalten: den positiven Spitzenwert der oszillierenden Welle, den negativen Spitzenwert der oszillierenden Welle, die Strecke, die das mit der oszillierenden Welle assoziierte Gammaphoton zurücklegt, um die Photorezeptorzellenanordnung zu erreichen, und die Zeit, die das Gammaphoton benötigt, um von der Photorezeptorzanordnung detektiert zu werden, wobei ein Erstellen (408) des Konturdiagramms ein Erstellen (408) des Konturdiagramms auf einem Millimetergraphen umfasst, umfassend einen positiv-positiven Quadranten, wobei positive Spitzen von mindestens einer der Vielzahl von oszillierenden Wellen in einer oberen Ebene des positiv-positiven Quadranten aufgetragen werden und negative Spitzen von mindestens einer der Vielzahl von oszillierenden Wellen in einer unteren Ebene des positiv-positiven Quadranten aufgetragen werden; und
Vergleichen (412), durch die Krebsidentifizierungsvorrichtung, eines Musters des Konturdiagramms mit einer Vielzahl von Trainingskonturdiagrammen, um eine Krebsart zu identifizieren, die mindestens einen Körperteil des Organismus betrifft, wobei jedes der Vielzahl von Trainingskonturdiagrammen mit einer assoziierten Krebsart markiert ist.

7. Verfahren nach Anspruch 6, ferner umfassend ein Trainieren der Krebsidentifizierungsvorrichtung zum Identifizieren des Krebstyps, indem eine Diagnose von Konturdiagrammen, die für eine Vielzahl von Organismen erstellt werden, von mindestens einem Arzt empfangen wird.

8. Computerlesbares Speichermedium, auf dem Anweisungen gespeichert sind, die, wenn sie von einem oder Vielzahl von Prozessoren ausgeführt werden, eine Krebsidentifizierungsvorrichtung veranlassen, das Verfahren nach Anspruch 6 oder Anspruch 7 durchzuführen.

## Revendications

1. Dispositif d'identification de cancer comprenant :
un réseau de cellules photoréceptrices (202) comprenant une pluralité de cellules photoréceptrices (202a à 2021) et conçu pour recevoir les photons gamma émis par les cellules cancéreuses présentes dans un organisme ;
un moyen (206) destiné à convertir les photons gamma en une pluralité de signaux électriques ;
un convertisseur analogique-numérique (212) destiné à convertir la pluralité de signaux électriques en une pluralité de signaux numériques ;
un oscillateur (214) destiné à convertir la pluralité de signaux numériques en la pluralité d'ondes oscillantes ;
un processeur couplé en communication au réseau de cellules photoréceptrices ; et
une mémoire (216) couplée en communication au processeur et sur laquelle sont stockées des instructions de processeur, qui, lors de leur exécution par le processeur, amènent le processeur à :
créer (306) un tracé de contour d'une fonction des pics positifs et négatifs de chacune de la pluralité d'ondes oscillantes, ladite fonction comprenant une fonction prédéfinie d'un ou plusieurs paramètres qui comprennent : la valeur de pic positif de l'onde oscillante, la valeur de pic négatif de l'onde oscillante, la distance parcourue par le photon gamma associé à l'onde oscillante pour atteindre le réseau de cellules photoréceptrices et le temps mis par le photon gamma pour être détecté par le réseau de photorécepteurs, lesdites instructions, lors de leur exécution, amenant le processeur à créer (306) le tracé de contour sur un graphique millimétrique comprenant un quadrant positif-positif, lesdits pics positifs d'au moins l'une de la pluralité d'ondes oscillantes étant tracés dans un plan supérieur du quadrant positif-positif et lesdits pics négatifs d'au moins l'une de la pluralité d'ondes oscillantes étant tracés dans un plan inférieur du quadrant positif positif ; et
comparer (308) un motif du tracé de contour à une pluralité de tracés de contour d'apprentissage pour identifier un type de cancer touchant au moins une partie du corps de l'organisme, chacun de la pluralité de tracés de contour d'apprentissage étant étiqueté avec un type de cancer associé.

2. Dispositif d'identification de cancer selon l'une quelconque des revendications précédentes, lesdites instructions de processeur, lors de leur exécution, amenant en outre le processeur à transformer (410) le tracé de contour en une vue d'image matricielle pour convertir le tracé de contour en valeur de pixel.

3. Dispositif d'identification de cancer selon l'une quelconque des revendications précédentes, lesdites instructions de processeur, lors de leur exécution, amenant en outre le processeur à entraîner le dispositif d'identification de cancer à identifier un type de cancer en recevant un diagnostic de tracés de contour créés pour une pluralité d'organismes provenant d'au moins un médecin.

4. Dispositif d'identification de cancer selon l'une quelconque des revendications précédentes, comprenant en outre un projecteur d'ondes oscillantes conçu pour créer le tracé de contour, ledit tracé de contour représentant le contour de ladite au moins une partie du corps touchée par le cancer.

5. Dispositif d'identification de cancer selon l'une quelconque des revendications précédentes, lesdites cellules photoréceptrices (202a à 202l) étant conçues pour recevoir des photons gamma émis suite à l'introduction de traceurs radioactifs dans l'organisme, lesdits traceurs radioactifs marquant les cellules cancéreuses et émettant des photons gamma.

6. Procédé implémenté par ordinateur permettant d'identifier un cancer comprenant :
la conversion des photons gamma en une pluralité de signaux électriques, lesdits photons gamma émis par des cellules cancéreuses présentes dans un organisme, par l'intermédiaire d'au moins une cellule photoréceptrice, ayant été reçus par un dispositif d'identification de cancer,
la conversion de la pluralité de signaux électriques en une pluralité de signaux numériques à l'aide d'un convertisseur analogique-numérique ;
la conversion de la pluralité de signaux numériques en la pluralité d'ondes oscillantes à l'aide d'un oscillateur ;
la création (408), par le dispositif d'identification de cancer, d'un tracé de contour d'une fonction des pics positifs et négatifs de chacune de la pluralité d'ondes oscillantes, ladite fonction comprenant une fonction prédéfinie d'un ou plusieurs paramètres qui comprennent : la valeur de pic positif de l'onde oscillante, la valeur de pic négatif de l'onde oscillante, la distance parcourue par les photons gamma associée à l'onde oscillante pour atteindre le réseau de cellules photoréceptrices et le temps mis par le photon gamma pour être détecté par le réseau photorécepteur, ladite création (408) du tracé de contour comprenant la création (408) du tracé de contour sur un graphique millimétrique comprenant un quadrant positifpositif, lesdits pics positifs d'au moins l'une de la pluralité d'ondes oscillantes étant tracés dans un plan supérieur du quadrant positif-positif et lesdits pics négatifs d'au moins l'une de la pluralité d'ondes oscillantes étant tracés dans un plan inférieur du quadrant positif positif ; et
la comparaison (412), par le dispositif d'identification de cancer, du motif du tracé de contour à une pluralité de tracés de contour d'apprentissage pour identifier le type de cancer touchant au moins une partie du corps de l'organisme, chacun de la pluralité de tracés de contour d'apprentissage étant étiqueté avec un type de cancer associé.

7. Procédé selon la revendication 6, comprenant en outre l'entraînement du dispositif d'identification de cancer à identifier le type de cancer en recevant un diagnostic de tracés de contour créés pour une pluralité d'organismes provenant d'au moins un médecin.

8. Support de stockage lisible par ordinateur sur lequel sont stockées des instructions qui, lorsqu'elles sont exécutées par un ou plusieurs processeurs, amènent un dispositif d'identification de cancer à exécuter le procédé selon la revendication 6 ou la revendication 7.
